(19) Europäisches Patentamt
European Patent Office
Office européen des brevets

(11) Veröffentlichungsnummer : **0 352 504 B1**

(12) # EUROPÄISCHE PATENTSCHRIFT

(45) Veröffentlichungstag der Patentschrift :
**16.09.92 Patentblatt 92/38**

(51) Int. Cl.5 : **C07C 255/12,** C07C 255/13, C07C 255/24

(21) Anmeldenummer : **89112043.8**

(22) Anmeldetag : **01.07.89**

(54) **Verfahren zur gemeinschaftlichen Herstellung von 3-Dialkylaminopropionitrilen, Bis-(2-cyanoethyl)-ether und gewünschtenfalls Ethylencyanhydrin.**

(30) Priorität : **23.07.88 DE 3825119**

(43) Veröffentlichungstag der Anmeldung :
**31.01.90 Patentblatt 90/05**

(45) Bekanntmachung des Hinweises auf die Patenterteilung :
**16.09.92 Patentblatt 92/38**

(84) Benannte Vertragsstaaten :
**BE DE ES FR GB IT NL**

(56) Entgegenhaltungen :
**EP-A- 0 206 298**
**US-A- 2 404 164**
**US-A- 2 425 693**
**US-A- 2 448 979**
**CHEMICAL ABSTRACTS, Band 8, 1968, Zusammenfassung Nr. 29272e, Columbus, Ohio, USA; SU-A-189 828**

(73) Patentinhaber : **BASF Aktiengesellschaft**
**Carl-Bosch-Strasse 38**
**W-6700 Ludwigshafen (DE)**

(72) Erfinder : **Merger, Franz, Dr.**
**Max-Slevogt-Strasse 25**
**W-6710 Frankenthal (DE)**
Erfinder : **Harder, Wolfgang, Dr.**
**Bergwaldstrasse 16**
**W-6940 Weinheim (DE)**
Erfinder : **Hettinger, Peter, Dr.**
**Sievertstrasse 4**
**W-6802 Ladenburg (DE)**
Erfinder : **Priester, Claus-Ulrich, Dr.**
**Ungsteiner Strasse 18**
**W-6700 Ludwigshafen (DE)**
Erfinder : **Franz, Dieter, Dr.**
**Horst-Schork-Strasse 75**
**W-6700 Ludwigshafen (DE)**
Erfinder : **Voges, Dieter, Dr.**
**Speyerer Strasse 113**
**W-6800 Mannheim 1 (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft ein neues Verfahren zur gemeinschaftlichen Herstellung von 3-Dialkylaminopropionitrilen der allgemeinen Formel I

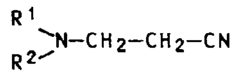

I

in der $R^1$ und $R^2$ $C_1$-$C_4$-Alkylgruppen bedeuten, die auch zu einem 5- oder 6-gliedrigen Ring verbunden sein können, Bis-(2-cyanoethyl)-ether II

$$NC\text{-}CH_2\text{-}CH_2\text{-}O\text{-}CH_2\text{-}CH_2\text{-}CN \qquad II$$

und gewünschtenfalls Ethylencyanhydrin III

$$HO\text{-}CH_2\text{-}CH_2\text{-}CN \qquad III$$

Die Verbindungen I bis III sind als Zwischenprodukte von großem Interesse. Dialkylaminopropionitrile I werden zur Herstellung von Desinfektionsmitteln, Netzmitteln und Weichmachern verwendet. Bis-(2-cyanoethyl)-ether II ist als Lösungsmittel z.B. für Celluloselacke wichtig, dient zur Aromatenextraktion aus Rohöl und ist ein Zwischenprodukt für die Herstellung von Weichmachern für PVC. Ethylencyanhydrin III findet vielseitige Verwendung als Zwischenprodukt für die Herstellung von Farbstoffen, Pflanzenschutzmitteln, Pharmaka und Kunststoffen.

Die Herstellung der einzelnen Produkte I, II und III ist bekannt. 3-Dialkyl-aminopropionitrile I werden im allgemeinen durch Addition von Dialkylaminen an Acrylnitril hergestellt (US-A 2 459 062, US-A 2 459 088). Auf diese Weise sind die Nitrile I problemlos zu gewinnen.

Demgegenüber sind bei der industriellen Herstellung von Bis-(2-cyanoethyl)-ether II eine Reihe von Schwierigkeiten zu überwinden. Verbindung II bildet sich in Gegenwart von Basen durch die Addition von einem Wassermolekül an zwei Moleküle Acrylnitril entsprechend Gleichung (1) (US-A 2 448 979).

$$2\ NC{-}CH{=}CH_2 + H_2O \longrightarrow \underset{II}{NC{-}CH_2{-}CH_2{-}O{-}CH_2{-}CH_2{-}CN} \qquad (1)$$

Diese Reaktion führt jedoch zur Bildung brauner Nebenprodukte, welche nicht oder nur sehr schwierig abzutrennen sind und den wert des Produktes stark mindern. Zur Behebung dieser Schwierigkeiten wurden eine Reihe von Verfahren entwickelt, welche jedoch durchweg stark mit Mängeln behaftet sind.

In der DE-B 11 89 975 ist ein Verfahren beschrieben, bei dem Acrylnitril in Gegenwart eines 100 bis 400 mol-%igen Wasserüberschusses zum Ether II umgesetzt wird. Da das Acrylnitril in diesem Verfahren nur zu 83 % umgesetzt wird, muß es destillativ zurückgewonnen und in den Prozeß zurückgeführt werden. Die Reinigung des Ethers 11 gestaltet sich außerdem sehr aufwendig, da bei der Destillation Fraktionen anfallen, die zurückgeführt werden müssen, und die Raum-Zeit-Ausbeute ist gering. Dieses Verfahren ist folglich unwirtschaftlich.

Nach der US-A 2 816 130 wird zur Herstellung des Ethers 11 ein Molverhältnis Acrylnitril-wasser von mindestens 4:1 angewandt. Davon abgesehen, daß dieses Verfahren aufgrund seiner schlechten Ausbeute (50 % d.Th.) indiskutabel ist, hat es ebenfalls den Nachteil, daß große Mengen wäßriger Acrylnitrillösung destillativ aufgearbeitet werden müssen - ein Nachteil, der ebenfalls den bisherigen Verfahren zur Herstellung von II anhaftet. Die Handhabung als auch die destillative Aufarbeitung dieser Acrylnitrillösungen sind - außer den zusätzlichen Kosten, welche durch sie verursacht werden - insofern problematisch, als das Acrylnitril toxikologisch nicht unbedenklich ist und außerdem im Zuge der Destillation zur Polymerisation neigt.

Zur Darstellung von Ethylencyanhydrin III eignet sich die Spaltung des Bis-(2-cyanoethyl)-ethers II. Dazu wird der Ether II nach JP-A 85 550/83 bei Temperaturen von 120 bis 200°C in Gegenwart schwacher Basen wie Tetraethylammoniumacetat in Ethylencyanhydrin III und Acrylnitril gespalten. Die verlustfreie wiedergewinnung des zur Polymerisation neigenden Acrylnitrils bereitet bei diesem Verfahren jedoch erhebliche Mühe.

Nach der DE-A 35 22 906 kann der Ether II mit Methanolaten zu Ethylencyanhydrin III und 3-Methoxypropionitril umgesetzt werden. Dieses Verfahren liefert zwar gute Ausbeuten, jedoch fallen dabei zwangsweise große Mengen 3-Methoxypropionitril an, welches nur in beschränktem Umfang Verwendung findet.

US-A 2 425 693 betrifft ein Verfahren zur Herstellung von β-Aminopropionitrilen durch die Umsetzung von β-Alkoxypropionitrilen mit aliphatischen Aminen bei Temperaturen von 150 bis 230°C.

Der Erfindung lag nun die Aufgabe zugrunde, ein Verfahren zur gemeinschaftlichen Herstellung von 3-Dialkylaminopropionitril I und Bis-(2-cyanoethyl)-ether II zu finden, das frei von den geschilderten Mängeln und Nachteilen ist, das insbesondere die destillative Aufarbeitung von überschüssigem oder nicht umgesetztem Acrylnitril unnötig macht und das gewünschtenfalls die Herstellung von Ethylencyanhydrin III aus Bis-(2-cyanoethyl)-ether II erlaubt, ohne daß diese mit den Nachteilen der bisherigen Verfahren behaftet ist.

Demgemäß wurde ein Verfahren zur gemeinschaftlichen Herstellung von 3-Dialkylaminopropionitrilen der allgemeinen Formel I

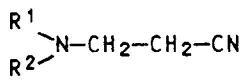 I,

in der $R^1$ und $R^2$ $C_1$-$C_4$-Alkylgruppen bedeuten, die auch zu einem 5- oder 6-gliedrigen Ring verbunden sein können, Bis-(2-cyanoethyl)-ether II

$NC-CH_2-CH_2-O-CH_2-CH_2-CN$ II

und gewünschtenfalls Ethylencyanhydrin III

$HO-CH_2-CH_2-CN$ III

gefunden,

das dadurch gekennzeichnet ist, daß man

a) Acrylnitril und Wasser in Gegenwart einer Base zu einem Gemisch aus Acrylnitril, Wasser und Bis-(2-cyanoethyl)-ether II umsetzt, wobei die Base eine Mineralbase, eine quartäre Stickstoffbase oder ein Gemisch aus diesen sein kann,

b) dieses Gemisch bei etwa 0 bis 50°C mit einem Dialkylamin der allgemeinen Formel IV

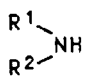 IV

zu einem Gemisch aus Wasser, Dialkylaminopropionitril I und Bis-(2-cyanoethyl)-ether II umsetzt und dieses Gemisch in seine Komponenten zerlegt und

c) gewünschtenfalls den in Verfahrensstufe b) erhaltenen Ether II oder eine wäßrige Lösung dieses Ethers und/oder die diesen Ether enthaltenden Reaktionsausträge aus den Verfahrensstufen a) oder b) bei Temperaturen von 50 bis 150°C mit dem Dialkylamin IV zu einem Gemisch aus 3-Dialkylaminopropionitril I und Ethylencyanhydrin III reagieren läßt und dieses gewünschtenfalls in seine Komponenten zerlegt.

In Verfahrensstufe a) wird Acrylnitril mit Wasser nach Gleichung (1) zu Bis-(2-cyanoethyl)-ether II umgesetzt. Diese Reaktion wird durch Basen katalysiert.

Als Basen verwendet man im allgemeinen wäßrige Lösungen von Alkalimetallhydroxiden, insbesondere Natrium- und Kaliumhydroxid, Alkalimetallcarbonaten, insbesondere Natrium- und Kaliumcarbonat und Erdalkalimetallhydroxiden. Besonders bevorzugte Basen sind wäßrige Lösungen von quartären Ammoniumhydroxiden wie Tetramethylammoniumhydroxid, Tetraethylammoniumhydroxid, Tetrapropylammoniumhydroxid, Tetrabutylammoniumhydroxid, Tetrapropylammoniumhydroxid, Dimethyl-bis-(hydroxyethyl)-ammoniumhydroxid und Tetrakis-(hydroxyethyl)-ammoniumhydroxid. Es können auch quartäre Ammoniumhydroxide verwendet werden, die langkettige Alkylreste oder Benzylreste tragen oder auch Gemische aus quartären Ammoniumhydroxiden und Mineralbasen. Ebenso ist es möglich, die quartären Ammoniumhydroxide im Reaktionsmedium aus den entsprechenden Ammoniumsalzen durch Zugabe von Alkalimetall- oder Erdalkalimetallhydroxiden zu erzeugen. Die Base kann in katalytischen Mengen eingesetzt werden, die Anwendung stöchiometrischer oder überschüssiger Mengen ist ebenfalls möglich. Bevorzugt wird die Base in katalytischen Mengen eingesetzt, und zwar in einem Verhältnis zu Acrylnitril von 1 bis 0,001, insbesondere von 1 bis 0,01 mol-%.

Zur Erzielung eines hohen Umsatzes und einer hohen Ausbeute an Bis-(2-cyanoethyl)-ether II können stöchiometrische oder überschüssige Mengen an Wasser zugesetzt werden. Zweckmäßig werden pro Mol Acrylnitril 0,5 bis 3,0 mol, vorteilhaft 0,8 bis 2 mol Wasser verwendet. Der Zusatz von inerten organischen Lösungsmitteln, wie z.B. Dioxan, Tetrahydrofuran oder Ethylenglykoldimethylether ist möglich.

Im allgemeinen erfolgt die Umsetzung von Acrylnitril und Wasser in Gegenwart von Basen bei Temperaturen von 60 bis 150°C. Entsprechend der gewählten Temperatur wird die Reaktion unter Atmosphärendruck (60 bis 85°C) oder unter Eigendruck (80 bis 150°C) vorgenommen. Temperaturen im Bereich von 80 bis 150°C,

insbesondere Temperaturen von 90 bis 140°C werden bevorzugt. Zur Durchführung der Reaktion kann sowohl die diskontinuierliche als auch die kontinuierliche Verfahrensweise gewählt werden. Wird die Umsetzung diskontinuierlich ausgeführt, so ist das Arbeiten unter Atmosphärendruck zweckmäßig, wogegen beim kontinuierlichen Verfahren die Druckfahrweise bevorzugt ist. Vorteilhaft wird die Umsetzung kontinuierlich unter erhöhtem Druck vorgenommen.

Bei der kontinuierlichen Durchführung des Verfahren können Rührreaktorkaskaden oder bevorzugt Rohrreaktoren zur Anwendung gelangen. Bei der Verwendung von Rührreaktorkaskaden können Verweilzeiten von 15 min bis 6 h eingestellt werden; bevorzugt sind Verweilzeiten von 30 min bis 3 h. Führt man die Reaktion in Rohrreaktoren durch, so wählt man eine Verweilzeit des Reaktionsgemisches von zweckmäßig 2 bis 45 min, vorteilhaft von 3 bis 30 min.

Bei der diskontinuierlichen Umsetzung legt man im allgemeinen das Acrylnitril vor, erwärmt auf 60 bis 70°C, fügt unter Erhitzen die wäßrige Basenlösung zu und hält das Reaktionsgemisch so lange auf der gewünschten Reaktionstemperatur bis der gewünschte Umsatz erreicht ist. Zweckmäßigerweise setzt man sowohl im diskontinuierlichen als auch im kontinuierlichen Verfahren nur 60 bis 90 Gew.%, vorzugsweise 70 bis 85 % des eingesetzten Acrylnitrils zu Bis-(2-cyanoethyl)-ether II um, da bei höheren Umsätzen mit einer verstärkten Nebenproduktbildung, bedingt durch Hydrolysereaktionen, zu rechnen ist.

Die nach diesem Verfahren erhaltenen Reaktionsgemische können bis zu 5 Gew.% Ethylencyanhydrin enthalten.

Zur Gewinnung des Wertproduktes 3-Dialkylaminopropionitril wird erfindungsgemäß der Reaktionsaustrag aus Verfahrensstufe a), der neben Acrylnitril und Bis-(2-cyanoethyl)-ether I noch Wasser und geringe Mengen an Ethylencyanhydrin enthält, ohne weitere Aufarbeitung in die Verfahrensstufe b) eingesetzt.

In der Verfahrensstufe b) wird das restliche Acrylnitril nach Gleichung (2) mit einem Dialkylamin IV zu 3-Dialkylaminopropionitril I umgesetzt.

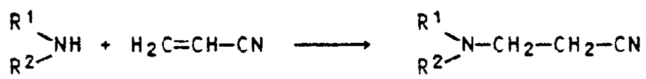 (2)

IV I

Dazu wird im allgemeinen der Reaktionsaustrag aus Verfahrensstufe a) bei Temperaturen von 0 bis 50°C, bevorzugt 15 bis 25°C, unter Kühlung mit vorzugsweise molaren Mengen an Dialkylamin IV, bezogen auf Acrylnitril, versetzt. Unter diesen Bedingungen wird das Acrylnitril praktisch quantitativ innerhalb kurzer Zeit in Dialkylaminopropionitril I umgewandelt. Dieser Verfahrensschritt kann diskontinuierlich in Rührreaktoren, aber auch kontinuierlich z.B. in Rührreaktorkaskaden oder bevorzugt in Rohrreaktoren ausgeführt werden.

Für das Verfahren sind prinzipiell alle $C_1$- bis $C_4$-Dialkylamine mit gleichen oder verschiedenen Resten $R^1$ und $R^2$ geeignet. Beispielhaft seien genannt: Dimethylamin, Diethylamin, Dipropylamin, Diisopropylamin, Methylethylamin, Dibutylamin, Diisobutylamin. Es können auch sekundäre Amine verwendet werden, bei denen $R^1$ und $R^2$ gemeinsam einen 5- bis 6-gliedrigen Ring bilden. Dimethylamin und Diethylamin werden bevorzugt eingesetzt.

Die Dialkylamine IV können sowohl unverdünnt - gasförmig oder verflüssigt - als auch in Form von Lösungen, vorzugsweise wäßrigen Lösungen, zur Verwendung gelangen.

Zur Aufarbeitung des Reaktionsgemisches geht man zweckmäßig so vor, daß man das 3-Dialkylaminopropionitril I gemeinsam mit dem Wasser und dem Nebenprodukt Ethylencyanhydrin III z.B. mit Hilfe eines Dünnschichtverdampfers abtrennt. Anschließend wird das verbleibende hochsiedende Gemisch mit einer verdünnten Mineralsäure neutralisiert und daraus der Bis-(2-cyanoethyl)-ether II durch fraktionierte Destillation unter vermindertem Druck isoliert.

Ein besonderer Vorteil des erfindungsgemäßen Verfahrens besteht darin, daß es leicht in der Weise abwandelbar ist, daß neben 3-Dialkylaminopropionitril I Ethylencyanhydrin III anstelle von Bis-(2-cyanoethyl)-ether II gewonnen werden kann.

Bei dieser Ausgestaltung des erfindungsgemäßen Verfahren wird der im Reaktionsaustrag der Verfahrensstufen a) oder b) vorliegende Bis-(2-cyanoethyl)-ether II ohne vorhergehende Isolierung im Temperaturbereich von etwa 50 bis 150°C mit einem Dialkylamin IV, entsprechend Gleichung (3) zum 3-Dialkylaminopropionitril I und Ethylencyanhydrin III umgesetzt.

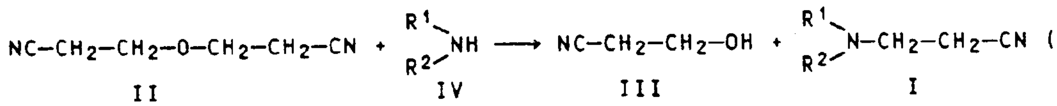

Setzt man den Reaktionsaustrag aus Verfahrensstufe a) in diese Verfahrensstufe ein, so reagiert das darin enthaltene Acrylnitril unter den Bedingungen der Etheraminolyse und einhergehend mit dieser, entsprechend Gleichung (2), mit dem Dialkylamin IV zum 3-Dialkylaminopropionitril ab. Natürlich kann auch der reine Bis-(2-cyanoethyl)-ether II oder eine ihn enthaltende Lösung als Edukt in diesem Verfahrensschritt dienen.

Im allgemeinen verwendet man 1 bis 3 mol, bevorzugt 1 bis 1,5 Mol Dialkylamin IV pro Mol Bis-(2-cyanoethyl)-ether II. Enthält die Reaktionsmischung noch Acrylnitril, so fügt man zusätzlich die zur Umsetzung des Acrylnitrils zu 3-Dialkylaminopropionitril I erforderliche Menge an Dialkylamin IV hinzu. Als Dialkylamine IV können alle in der Beschreibung der Verfahrensstufe b) angeführten Dialkylamine zur Anwendung gelangen und zwar sowohl unverdünnt als auch in Form einer Lösung, zweckmäßig als wäßrige Lösung. Bevorzugte Dialkylamine IV sind Dimethylamin und Diethylamin.

Die Aminolyse des Ethers II setzt bereits bei Temperaturen um 50°C ein. Doch ist in diesem Temperaturbereich die Reaktionsgeschwindigkeit so niedrig, daß Reaktionszeiten über 5 Stunden benötigt werden. Im allgemeinen führt man deshalb die Umsetzung bei Temperaturen oberhalb 50°C in einem Temperaturbereich bis zweckmäßig 150°C, vorzugsweise im Temperaturbereich von 80 bis 130°C, insbesondere bei 100 bis 130°C durch. Die Anwendung höherer Temperaturen als 150°C ist möglich, führt jedoch zu keinen weiteren Vorteilen. Die Umsetzung kann diskontinuierlich in Rührreaktoren oder kontinuierlich in Rührreaktorkaskaden oder Rohrreaktoren, bei Atmosphärendruck oder under erhöhtem Druck ausgeführt werden.

Besonders bevorzugt setzt man den Reaktionsaustrag aus Verfahrensstufe a) in den angegebenen Temperaturbereichen, insbesondere bei 100 bis 130°C und Verweilzeiten von 0,5 bis 45 min, vorteilhaft 0,5 bis 5 Minuten, kontinuierlich und unter erhöhtem Druck in einem Rohrreaktor um.

Dabei sind mehrere Verfahrensweisen möglich.

Im allgemeinen kühlt man den Reaktionsaustrag aus Verfahrensstufe a) oder b) auf Temperaturen von 0 bis 30°C, vorzugsweise 15 bis 25°C ab und fügt diesem Reaktionsgemisch das entsprechende Dialkylamin IV zu. Da sowohl die Aminolyse des Bis-(2-cyanoethyl)-ethers II als auch die einhergehende Bildung des 3-Dialkylaminopropionitrils I aus restlichem Acrylnitril exotherm verläuft, erhöht sich bei Zugabe des Dialkylamins IV die Temperatur des Reaktionsgemisches von selbst auf die gewünschte Reaktionstemperatur. Um die für einen vollständigen Umsatz erforderlichen Verweilzeiten auf 0,5 bis 45 min zu verkürzen, ist es günstig, das Reaktionsgemisch nach Zugabe des Dialkylamins IV zunächst auf 50 bis 80°C zu erwärmen. Die Reaktion hält sich durch die gebildete Reaktionswärme selbst in Gange.

Bei dieser Arbeitsweise bietet es sich an, die Abkühlung der Reaktionsausträge a) oder b) und die Wiederaufheizung des Reaktionsgemisches nach Zugabe des Amins IV in einem Wärmeaustauscher vorzunehmen.

Der durch Zugabe des Amins IV im Reaktionsgemisch bewirkte Temperaturanstieg ist natürlich von der Zugabeform des Amins abhängig. Wird z.B. eine 40 gew.%ige wäßrige Dimethylamin-Lösung zugesetzt, so entspricht die freigesetzte Reaktionswärme in etwa der zum Aufheizen des Reaktionsgemisches auf die gewünschte Reaktionstemperatur erforderlichen Wärmemenge. Wird hingegen flüssiges, unverdünntes Dimethylamin zugegeben, so muß zusätzlich Wärme mit Hilfe von Kühlvorrichtungen in oder am Reaktor abgeführt werden, soll der gewünschte Temperaturbereich nicht überschritten werden.

Weiterhin ist es möglich, das Amin IV den Reaktionsausträgen aus Verfahrensstufe a) oder b) zuzuführen, ohne diese vorher abzukühlen. Wird diese Verfahrensweise auf den Reaktionsaustrag aus Verfahrensstufe a) angewendet, versteht es sich von selbst, daß in diesem Falle überschüssige Reaktionswärme durch Kühlung abgeführt werden muß.

Die Spaltung des Ethers II wird im allgemeinen bei Temperaturen von 50 bis 150°C, bevorzugt von 80 bis 150°C vorgenommen. Die Umsetzung kann diskontinuierlich in Rührreaktoren oder kontinuierlich in Rührreaktorkaskaden oder in Rohrreaktoren, bei Atmosphärendruck oder unter erhöhtem Druck ausgeführt werden. Besonders bevorzugt setzt man den Reaktionsaustrag aus Verfahrensstufe a) bei Temperaturen von 50 bis 150°C, vorteilhaft 100 bis 130°C und Verweilzeiten von 0,5 bis 45 min, vorteilhaft 0,5 bis 5 Minuten, kontinuierlich und unter erhöhtem Druck in einem Rohrreaktor um.

Der Reaktionsaustrag kann auf die übliche Art und Weise in seine Komponenten aufgetrennt werden. Da der Reaktionsaustrag nur sehr geringe Anteile an Verunreinigungen enthält, kann er gewünschtenfalls ohne weitere Reinigung weiterverarbeitet werden. Beispielsweise ist es möglich, die im Reaktionsaustrag enthaltenen Verbindungen 3-Dialkylaminopropionitril I und Ethylencyanhydrin III gemeinsam in einer Hydrierstufe zu

den entsprechenden Aminen zu hydrieren oder sie mit starken Mineralbasen zu den entsprechenden Carboxylaten zu verseifen.

Ethylencyanhydrin III kann nach diesem Verfahren neben 3-Dialkylaminopropionitril in guten Ausbeuten erhalten werden.

Beispiel 1

Zu 600 g (11,3 mol) Acrylnitril wurde bei 70°C innerhalb von 45 min eine Lösung aus 5,0 g (0,024 mol) Tetrakis-(hydroxyethyl)-ammoniumhydroxid und 163 g (9,0 mol) Wasser gegeben. Anschließend wurde diese Mischung 45 min bei 80 bis 83°C gerührt.

Nachdem das erhaltene Gemisch auf 15 bis 20°C abgekühlt worden war, wurde es unter Kühlung mit 260 g einer wäßrigen 40 %igen Dimethylaminlösung (2,3 mol) vermischt und 15 min gerührt.

Aus der so erhaltenen Produktmischung wurde mittels eines Dünnschichtverdampfers bei einer Temperatur von 90°C und einem Druck von 4 mbar ein Gemisch aus Wasser, 3-Dimethylaminopropionitril und Ethylencyanhydrin abgetrennt und anschließend durch fraktionierte Destillation in seine Komponenten zerlegt. Die im Sumpf des Dünnschichtverdampfers verbliebene hochsiedende Flüssigkeit wurde mit verdünnter Schwefelsäure auf einen pH-Wert von 6 eingestellt und anschließend bei vermindertem Druck fraktionierend destilliert. Es wurde die Fraktion im Siedebereich von 125 bis 140°C (Druck: 3 mbar) gesammelt.

Ausbeute (bez. auf Acrylnitril): 74,8 % Bis-(2-cyanoethyl)-ether
13,8 % 3-Dimethylaminopropionitril
7,6 % Ethylencyanhydrin

Beispiel 2

Zu 954 g (18 mol) Acrylnitril wurde bei 70°C innerhalb von 45 min eine Lösung von 1,85 g (0,009 mol) Tetrakis-(hydroxyethyl)-ammoniumhydroxid und 0,3 g (0,0075 mol) Natriumhydroxid in 261 g (14,5 mol) Wasser getropft. Anschließend wurde das Gemisch unter Rühren 75 min zum Sieden erhitzt.

Das erhaltene Reaktionsgemisch enthielt gemäß quantitativer gaschromatographischer Analyse 16 % Acrylnitril (Umsatz: 79,6 %), 5,6 % Ethylencyanhydrin und 68 % Bis-(2-cyanoethyl)-ether (Selektivität: 93,2 %).

Zu dieser Mischung wurden unter Kühlung auf 15 bis 20°C 1536 g einer 40 gew.%igen wäßrigen Dimethylamin-Lösung (13,7 mol) gegeben. 689 g der so erhaltenen Mischung wurden auf 80°C erhitzt und 50 min bei dieser Temperatur gerührt.

Nach beendeter Reaktion enthielt das Gemisch nach quantitativer gaschromatographischer Analyse 35,1 % 3-Dimethylaminopropionitril, 18 % Ethylencyanhydrin und 1,4 % Bis-(2-cyanoethyl)-ether. Die Gesamtausbeute an Ethylencyanhydrin und 3-Dimethylaminopropionitril betrug 93,6 % bezogen auf eingesetztes Acrylnitril.

Das Wasser wurde durch eine azeotrope Destillation mit Cyclohexan aus dem Produktgemisch entfernt und danach fraktioniert unter vermindertem Druck destilliert.

Ausbeute (bez. auf Acrylnitril): 49,2 % 3-Dimethylaminopropionitril
36,3 % Ethylencyanhydrin

Beispiel 3

Durch einen 23 ml Rohrreaktor wurden bei einer Temperatur von 130°C und einem Druck von 6 bar stündlich 46,4 ml (0,71 mol) Acrylnitril und eine Lösung aus 0,28 g (0,0013 mol) Tetrakis-(hydroxyethyl)-ammoniumhydroxid und 23,3 g (1,3 mol) Wasser gepumpt.

400 g Reaktionsaustrag wurden bei 20°C unter Kühlung mit 263 g einer 40 gew.%igen wäßrigen Dimethylaminlösung (2,3 mol) versetzt und 1 h bei 80°C gerührt.

Das Produktgemisch wurde wie in Beispiel 2 beschrieben aufgearbeitet.

Ausbeute (bez. auf Acrylnitril): 44,8 % 3-Dimethylaminopropionitril
37,6 % Ethylencyanhydrin

Beispiel 4

Acrylnitril wurde analog Beispiel 2 zu Ethylencyanhydrin und Bis-(2-cyanoethyl)-ether umgesetzt, wonach die erhaltene Mischung mit einer 40 %igen wäßrigen Dimethylaminlösung versetzt wurde.

689 g der so erhaltenen Mischung wurden bei einer Temperatur von 130°C, einem Druck von 10 bar und einer mittleren Verweilzeit von 1 min durch einen 23 ml Rohrreaktor gepumpt.

Ausbeute (nach quantitativer gaschromatographischer Analyse bezogen auf Acrylnitril):
57 % 3-Dimethylaminopropionitril
39,2% Ethylencyanhydrin

Beispiel 5

Zu 212 g (4 mol) Acrylnitril wurde bei 70°C eine Lösung von 1,7 g (0,008 mol) Tetrakis-(hydroxyethyl)-ammoniumhydroxid in 58 g (3,2 mol) Wasser gegeben. Anschließend wurde dieses Gemisch für 1 h auf 82°C erhitzt. Das Reaktionsgemisch wurde auf 5°C abgekühlt, 99 g (2,2 mol) flüssiges Dimethylamin unter Kühlung hinzugefügt und die entstandene Mischung bei 130°C und einer mittleren Verweilzeit von 3 min durch einen 2,2 ml Rohrreaktor gepumpt.

Der Reaktionsaustrag wurde durch eine fraktionierte Destillation aufgearbeitet.

Ausbeute (bez. auf Acrylnitril): 48,9 % N,N-Dimethylaminopropionitril
33,8 % Ethylencyanhydrin

Beispiel 6

Durch eine Kaskade aus zwei Glasautoklaven mit einem Volumen von 315 bzw. 320 ml wurden bei einer Temperatur von 85°C und einem Druck von 6 bar unter intensivem Rühren stündlich 706,2 ml (10,8 mol) Acrylnitril und eine Lösung aus 4,35 g (0,021 mol Tetrakis-(hydroxyethyl)-ammoniumhydroxid und 149,4 g (8,3 mol) Wasser gepumpt und anschließend auf Atmosphärendruck entspannt.

Der Austrag wurde durch einen Wärmetauscher geleitet, bei einer Temperatur von 20°C stündlich mit 541 g (7.4 mol) Diethylamin versetzt und wieder auf eine Temperatur von 70 bis 85°C erwärmt. Nach Verlassen des Wärmetauschers wurde das Reaktionsgemisch noch 1,5 h in einer weiteren Kaskade bei 70 bis 85°C gehalten. Der Reaktionsaustrag wurde durch fraktionierende Destillation aufgearbeitet.

Ausbeute (bez. auf Acrylnitril): 52,3 % 3-Diethylaminopropionitril
28,6 % Ethylencyanhydrin

Beispiel 7

Durch einen 23 ml-Rohrreaktor wurden bei einer Temperatur von 100°C und einem Druck von 10 bar stündlich 114 ml (92,3 g; 1,74 mol) Acrylnitril und eine Lösung von 0,84 g (0,004 mol) Tetrakis-(2-hydroxyethyl)-ammoniumhydroxid in 29,2 g (1,6 mol) Wasser gepumpt und anschließend in einem Wärmetauscher auf 20°C abgekühlt. Dazu wurden kontinuierlich 199,8 ml/h (177,8 g/h) einer 40 %igen wäßrigen Dimethylaminlösung (entsprechend 71,1 g; 1,58 mol Dimethylamin) zudosiert und das Gemisch in einem zweiten Rohrreaktor mit einem Volumen von 8,5 ml bei einer Temperatur von 130°C, einem Druck von 10 bar und einer mittleren Verweilzeit von 1,5 Minuten umgesetzt.

Der Reaktionsaustrag wurde wie in Beispiel 2 beschrieben aufgearbeitet.

Ausbeute (bez. auf Acrylnitril): 38 % Ethylencyanhydrin
56 % 3-Dimethylaminopropionitril.

## Patentansprüche

1. Verfahren zur gemeinschaftlichen Herstellung von 3-Dialkylaminopropionitrilen der allgemeinen Formel I

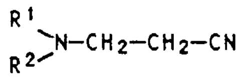

I,

in der $R^1$ und $R^2$ $C_1$-$C_4$-Alkylgruppen bedeuten, die auch zu einem 5- oder 6-gliedrigen Ring verbunden sein können, Bis-(2-cyanoethyl)-ether II

$$NC\text{-}CH_2\text{-}CH_2\text{-}O\text{-}CH_2\text{-}CH_2\text{-}CN \qquad II$$

und gewünschtenfalls Ethylencyanhydrin III

$$HO\text{-}CH_2\text{-}CH_2\text{-}CN \qquad III,$$

dadurch gekennzeichnet, daß man

a) Acrylnitril und Wasser in Gegenwart einer Base zu einem Gemisch aus Acrylnitril, Wasser und Bis-(2-cyanoethyl)-ether II umsetzt, wobei die Base eine Mineralbase, eine quartäre Stickstoffbase oder ein Gemisch aus diesen sein kann,

b) dieses Gemisch bei etwa 0 bis 50°C mit einem Dialkylamin der allgemeinen Formel IV

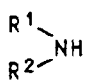

IV

zu einem Gemisch aus Wasser, Dialkylaminopropionitril I und Bis-(2-cyanoethyl)-ether II umsetzt und dieses Gemisch in seine Komponenten zerlegt und

c) gewünschtenfalls den in Verfahrensstufe b) erhaltenen Ether II oder eine wäßrige Lösung dieses Ethers und/oder die diesen Ether enthaltenden Reaktionsausträge aus den Verfahrensstufen a) oder b) bei Temperaturen von 50 bis 150°C mit dem Dialkylamin IV zu einem Gemisch aus 3-Dialkylaminopropionitril I und Ethylencyanhydrin III reagieren läßt und dieses gewünschtenfalls in seine Komponenten zerlegt.

2. Verfahren nach Anspruch 1, dadurch gekennzeichnet, daß man es kontinuierlich unter Druck in Rohrreaktoren durchführt und dabei für die Verfahrensstufe a) eine Verweilzeit von 2 bis 45 Minuten und für die Verfahrensstufen b) und c) Verweilzeiten von 0,5 bis 45 Minuten wählt.

3. Verfahren nach den Ansprüchen 1 und 2, dadurch gekennzeichnet, daß man den Reaktionsaustrag aus der Verfahrensstufe a) vor der Überführung in Verfahrensstufe c) auf 0 bis 30°C abkühlt, dann das Dialkylamin IV zuführt und die entstehende Reaktionswärme zur Erhitzung des Reaktionsgemisches auf 50 bis 150°C nutzt.

4. Verfahren nach den Ansprüchen 1 bis 3, dadurch gekennzeichnet, daß man den Reaktionsaustrag aus den Verfahrensstufen a) oder b) vor der Überführung in Verfahrensstufe c) auf 0 bis 30°C abkühlt, dann das Dialkylamin IV zuführt, anschließend das Reaktionsgemisch auf 50 bis 80°C erwärmt und die entstehende Reaktionswärme zur weiteren Erhitzung des Reaktionsgemisches nutzt.

5. Verfahren nach den Ansprüchen 1 bis 4, dadurch gekennzeichnet, daß man den Reaktionsaustrag aus den Verfahrensstufen a) oder b) vor der Überführung in Verfahrensstufe c) auf Temperaturen von 20 bis 25°C abkühlt.

6. Verfahren nach den Ansprüchen 1 und 2, dadurch gekennzeichnet, daß man den Reaktionsaustrag aus den Verfahrensstufen a) oder b) ohne vorherige Abkühlung mit dem Dialkylamin IV zusammenführt.

## Claims

1. A process for the joint preparation of a 3-dialkylaminopropionitrile of the formula I

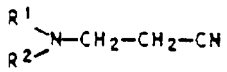

I

where $R^1$ and $R^2$ are each $C_1$-$C_4$-alkyl which may furthermore be bonded to form a 5-membered or 6-membered ring, bis-(2-cyanoethyl) ether II

$NC\text{-}CH_2\text{-}CH_2\text{-}O\text{-}CH_2\text{-}CH_2\text{-}CN$ II

and, if desired, ethylenecyanohydrin III

$HO\text{-}CH_2\text{-}CH_2\text{-}CN$ III

wherein

a) acrylonitrile and water are reacted in the presence of a base to give a mixture of acrylonitrile, water and bis-(2-cyanoethyl) ether II, the base being a mineral base, a quaternary nitrogen base or a mixture of these,

b) this mixture is reacted at about 0-50°C with a dialkylamine of the general formula IV

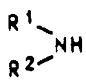

IV

to give a mixture of water, a dialkylaminopropionitrile I and bis-(2-cyanoethyl) ether II, and this mixture is separated into its components and

c) if desired, the ether II obtained in process stage b) or an aqueous solution of this ether and/or the reacted mixtures containing this ether and obtained from process stage a) or b) is allowed to react at from 50 to 150°C with the dialkylamine IV to give a mixture of a 3-dialkylaminopropionitrile I and ethylenecyanohydrin III, and, if desired, the said mixture is separated into its components.

2. A process as claimed in claim 1, which is carried out continuously under superatmospheric pressure in a tube reactor, and a residence time of from 2 to 45 minutes is chosen for process stage a) and residence times of from 0.5 to 45 minutes for process stages b) and c).

3. A process as claimed in claim 1 or 2, wherein the reacted mixture from process stage a) is cooled to 0-30°C before being transferred to process stage c), after which the dialkylamine IV is fed in and the resulting heat of reaction is used for heating the reaction mixture to 50-150°C.

4. A process as claimed in any of claims 1 to 3, wherein the reacted mixture from process stage a) or b) is cooled to 0-30°C before being transferred to process stage c), after which the dialkylamine IV is fed in and then the reaction mixture is heated to 50-80°C and the resulting heat of reaction is used for further heating of the reaction mixture.

5. A process as claimed in any of claims 1 to 4, wherein the reacted mixture from process stage a) or b) is cooled to 20-25°C before being transferred to process stage c).

6. A process as claimed in claim 1 or 2, wherein the reacted mixture from process stage a) or b) is combined with the dialkylamine IV without prior cooling.

## Revendications

1.- Procédé pour la préparation en commun de 3-dialkylaminopropionitriles de formule générale I

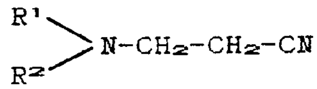 I

dans laquelle $R^1$ et $R^2$ représentent des groupements alkyle en $C_1$-$C_4$ qui peuvent être également reliés à un noyau penta- ou hexagonal,
de bis(2-cyanoéthyl)éther II

$NC-CH_2-CH_2-O-CH_2-CH_2-CN$ II

et, si on le désire, de cyanhydrine d'éthylène III

$HO-CH_2-CH_2-CN$ III

caractérisé en ce que

a) on fait réagir de l'acrylonitrile et de l'eau en présence d'une base, pour obtenir un mélange d'acrylonitrile, d'eau et de bis(2-cyanoéthyl)éther II, la base pouvant être une base minérale, une base azotée quaternaire ou un mélange de celles-ci,

b) on fait réagir ce mélange, à une température d'environ 0 à 50°C, avec une dialkylamine de formule générale IV

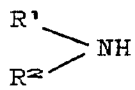 IV

pour obtenir un mélange d'eau, de dialkylaminopropionitrile I et de bis(2-cyanoéthyl)éther II et on décompose ce mélange en ses composants, et

c) on fait, si on le désire, réagir l'éther II obtenu dans l'étape b) ou une solution aqueuse de cet éther et/ou les produits réactionnels des étapes a) ou b), contenant cet éther, à des températures de 50 à 150°C, avec la dialkylamine IV, pour obtenir un mélange de 3-dialkylaminopropionitrile I et de cyanhydrine d'éthyléne III, et on décompose si on le désire ce mélange en ses composants.

2.- Procédé selon la revendication 1, caractérisé en ce qu'on l'exécute de façon continue sous pression dans des réacteurs tubulaires, en choisissant, pour l'étape b), une durée de séjour de 2 à 45 mn et, pour les étapes b) et c), des durées de séjour de 0,5 à 45 mn.

3.- Procédé selon la revendication 1 ou 2, caractérisé en ce qu'on refroidit à une température de 0 à 30°C le produit réactionnel de l'étape a) avant de l'envoyer dans l'étape c), puis on fait arriver la dialkylamine IV et on utilise la chaleur de réaction produite pour chauffer le mélange réactionnel à 50-150°C.

4.- Procédé selon l'une quelconque des revendications 1 à 3, caractérisé en ce qu'on refroidit à 0-30°C le produit réactionnel des étapes a) ou b) avant de l'envoyer dans l'étape c), puis on fait arriver la dialkylamine IV, on réchauffe ensuite le mélange réactionnel à 50-80°C et on utilise la chaleur de réaction produite pour chauffer davantage le mélange réactionnel.

5.- Procédé selon l'une quelconque des revendications 1 à 4, caractérisé en ce qu'on refroidit à des températures de 20 à 25°C le produit réactionnel des étapes a) ou b) avant de l'envoyer dans l'étape c).

6.- Procédé selon la revendication 1 ou 2, caractérisé en ce qu'on réunit le produit réactionnel des étapes a) ou b) à la dialkylamine IV sans refroidissement préalable.